# EUROPEAN PATENT SPECIFICATION

(11) **EP 3 353 553 B1**
(45) Date of publication and mention of the grant of the patent: **17.11.2021**
(21) Application number: 16849728.7
(22) Date of filing: 23.09.2016
(51) Int. Cl.: G01N 33/574

(54) **ANDROGEN RECEPTOR VARIANT 7 AS A BIOMARKER FOR TREATMENT SELECTION IN PATIENTS WITH METASTATIC CASTRATION RESISTANT PROSTATE CANCER (MCRPC)**
ANDROGENREZEPTORVARIANTE 7 ALS BIOMARKER FÜR DIE BEHANDLUNGSAUSWAHL BEI PATIENTEN MIT METASTASIERENDEM KASTRATIONSRESISTENTEM PROSTATAKREBS (MCRPC)
UTILISATION DU VARIANT 7 DU RÉCEPTEUR AUX ANDROGÈNES COMME BIOMARQUEUR POUR LA SÉLECTION THÉRAPEUTIQUE DE PATIENTS ATTEINTS D'UN CANCER MÉTASTATIQUE DE LA PROSTATE RÉSISTANT À LA CASTRATION (CPRCM)

(30) Priority: 25.09.2015 US 201562233206 P
(43) Date of publication of application: 01.08.2018
(73) Proprietor: Epic Sciences, Inc., San Diego, CA 92121 (US)
(72) Inventor: DITTAMORE, Ryan, San Diego, CA 92121 (US)
(74) Representative: Jones Day
(86) International application number: PCT/US2016/053387
(87) International publication number: WO 2017/053763

(56) References cited:
- US-A1- 2015 212 089
- US-A1- 2015 212 089
- US-A1- 2015 233 927
- EMMANUEL S. ANTONARAKIS ET AL: "Androgen Receptor Splice Variant 7 and Efficacy of Taxane Chemotherapy in Patients With Metastatic Castration-Resistant Prostate Cancer", JAMA ONCOLOGY, vol. 1, no. 5, 1 August 2015 (2015-08-01), page 582, XP055566931, US ISSN: 2374-2437, DOI: 10.1001/jamaoncol.2015.1341
- HOWARD I. SCHER ET AL: "Association of AR-V7 on Circulating Tumor Cells as a Treatment-Specific Biomarker With Outcomes and Survival in Castration-Resistant Prostate Cancer", JAMA ONCOLOGY, vol. 2, no. 11, 4 June 2016 (2016-06-04), page 1441, XP055387796, US ISSN: 2374-2437, DOI: 10.1001/jamaoncol.2016.1828
- YIEN NING SOPHIA WONG ET AL: "Evolution of androgen receptor targeted therapy for advanced prostate cancer", NATURE REVIEWS CLINICAL ONCOLOGY, vol. 11, no. 6, 20 May 2014 (2014-05-20), pages 365-376, XP055567743, NY, US ISSN: 1759-4774, DOI: 10.1038/nrclinonc.2014.72
- M CRESPO ET AL: "Androgen receptor expression in circulating tumour cells from castration-resistant prostate cancer patients treated with novel endocrine agents", BRITISH JOURNAL OF CANCER, vol. 112, no. 7, 26 February 2015 (2015-02-26), pages 1166-1174, XP055567023, GB ISSN: 0007-0920, DOI: 10.1038/bjc.2015.63

## Description

This application claims the benefit of U.S. Provisional Application No. 62/233,206, filed September 25, 2015.

The present disclosure relates generally to methods for selecting a therapy for a metastatic castration resistant prostate cancer (mCRPC) patient comprising detection of Androgen Receptor Variant 7 (AR-V7) in circulating tumor cells (CTCs).

### BACKGROUND

Prostate cancer (PC) remains the most common non-cutaneous cancer in the US. In 2014 alone, the projected incidence of prostate cancer is 233,000 cases with deaths occurring in 29,480 men, making metastatic prostate cancer therapy truly an unmet medical need. Siegel et al., 2014. CA Cancer J Clin. 2014;64(1):9―29. Epidemiological studies from Europe show comparable data with an estimated incidence of 416700 new cases in 2012, representing 22.8% of cancer diagnoses in men. In total, 92200 PC-specific deaths are expected, making it one of the three cancers men are most likely to die from, with a mortality rate of 9.5%

With the advent of exponential growth of novel agents tested and approved for the treatment of patients with metastatic castration-resistant prostate cancer (mCRPC) in the last 5 years alone, issues regarding the optimal sequencing or combination of these agents have arisen. Several guidelines exist that help direct clinicians as to the best sequencing approach and most would evaluate presence or lack of symptoms, performance status, as well as burden of disease to help determine the best sequencing for these agents. Mohler et al., 2014, J Natl Compr Canc Netw. 2013;11(12):1471―1479; Cookson et al. , 2013, J Urol. 2013;190(2):429-438. Currently, approved treatments consist of the taxane class of cytotoxic drugs and androgen-targeted therapies. The challenge for clinicians is to decide the best sequence for administering these therapies to provide the greatest benefit to patients. However, therapy failure remains a significant challenge based on heterogenous responses to therapies across patients and in light of cross-resistance from each agent. Mezynski et al. , Ann Oncol. 2012;23(11):2943-2947;. Noonan et al., Ann Oncol. 2013;24(7):1802―1807; Pezaro et al. , Eur Urol. 2014, 66( 3): 459―465. In addition, patients may lose the therapeutic window to gain substantial benefit from each drug that has been proven to provide overall survival gains. Hence, better methods of identifying the target populations who have the most potential to benefit from targeted therapies remain an important goal.

Circulating tumor cells (CTCs) represent a significant advance in cancer diagnosis made even more attractive by their non-invasive measurement. Cristofanilli et al., N Engl J Med 351:781-91, (2004) CTCs released from either a primary tumor or its metastatic sites hold important information about the biology of the tumor. Quantifying and characterizing CTCs, as a liquid biopsy, assists clinicians to select the course of therapy and to watch monitor how a patient's cancer evolves. CTCs can therefore be considered not only as surrogate biomarkers for metastatic disease but also as a promising key tool to track tumor changes, treatment response, cancer recurrence or patient outcome non-invasively. Historically, the extremely low levels of CTCs in the bloodstream combined with their unknown phenotype has significantly impeded their detection and limited their clinical utility. A variety of technologies are presently being developed developed for detection, isolation and characterization of CTCs in order to utilize their information.

Androgen receptor signaling directed therapies (AR Therapy), including Abiraterone Acetate + Prednisone (A) and Enzalutamide (E), prolong survival in patients with mCRPC and are FDA approved. The presence of the splice variant AR-V7 mRNA in EpCAM selected CTCs has been prospectively linked to resistance to A & E (Antonarakis et al. New England Journal of Medicine 371.11 (2014): 1028-1038) but not to taxane chemotherapy (T) (Antonarakis et al. J Clin Oncol 33, 2015 (suppl 7; abstr 138). AR-V7 may provide clinical utility in therapy selection between A & E or T.

A key limitation to the predictive value of an AR-V7 mRNA assay in CTCs is the analytical validation of the robustness of low frequency and labile mRNA measurement in CTCs, which may not meet diagnostic workflows amenable to community practices. Additionally, inability to assess EpCAM- CTCs may lead to undersampling the AR-V7 biomarker. There exists a need for a diagnostic test with clinical utility to inform the choice or guide the selection of A and E or T. The present invention addresses this need and provides related advantages.

### SUMMARY

The present disclosure describes an AR-V7 immunofluorescent test for use in fixed single CTCs, inclusive of all CTC subtypes utilizing samples from progressive mCRPC patients in need of a change in therapy.

The present invention is directed to a method of identifying an mCRPC patient with an improved response to taxane therapy compared to androgen receptor (AR) targeted therapy comprising (a) performing a direct analysis comprising immunofluorescent staining and morphological characterization of nucleated cells in a blood sample obtained from the patient to generate circulating tumor cell (CTC) data, wherein the direct analysis means the detection of the CTCs in the context of all surrounding nucleated cells present in the sample as opposed to after enrichment of the sample for CTCs prior to detection, and wherein the analysis comprises detecting the presence of an Androgen Receptor Variant 7 (AR-V7) in said cells, and (b) evaluating the CTC data to identify a mCRPC patient with an improved response to taxane therapy compared to Androgen receptor signaling-directed (ARS-directed) therapy, wherein the presence of an Androgen Receptor Variant 7 in said CTCs identifies a mCRPC patient with an improved response to taxane therapy compared to ARS-directed therapy.

In some embodiments, said mCRPC patient with an improved response to taxane therapy compared to ARS-directed therapy is identified based on nuclear localization of the AR-V7 in CTCs.

In some embodiments, the nuclear localization of the AR-V7 corresponds to resistance to ARS-directed therapy.

In some embodiments, the nuclear localization of the AR-V7 corresponds to an improved response to taxane therapy compared to ARS-directed therapy.

In some embodiments, the nuclear localization comprises a staining pattern with signal intensity ≥3-fold higher than background staining from neighboring white blood cells (WBCs).

The invention further provides a taxane for use in a method of treating metastatic castration resistant prostate cancer (mCRPC), wherein the method comprises identifying a mCRPC patient with an improved response to taxane therapy compared to androgen receptor signaling-directed (ARS-directed) therapy as defined in any one of claims 1 to 14; and treating the patient with taxane therapy if the patient has been identified as having an improved response to taxane therapy compared to ARS-directed therapy.

Disclosed is a method of performing a direct analysis comprising immunofluorescent staining and morphological characterization of nucleated cells in a blood sample obtained from a metastatic castration resistant prostate cancer (mCRPC) patient to generate circulating tumor cell (CTC) data, wherein the analysis comprises detecting the presence of an Androgen Receptor Variant 7 (AR-V7) in said cells.

Also disclosed is a method of performing a direct analysis comprising immunofluorescent staining and morphological characterization of nucleated cells in a blood sample obtained from a mCRPC patient to generate CTC data, wherein the analysis comprises detecting the presence of AR-V7 localized in the nucleus of CTCs.

Also disclosed is a method of performing a direct analysis comprising immunofluorescent staining and morphological characterization of nucleated cells in a blood sample obtained from a mCRPC patient to generate CTC data, wherein the analysis comprises detecting the presence of AR-V7 localized in the nucleus of CTCs, wherein the nuclear localization of the AR-V7 corresponds to resistance to ARS-directed therapy for the mCRPC patient.

Also disclosed is a method of performing a direct analysis comprising immunofluorescent staining and morphological characterization of nucleated cells in a blood sample obtained from a mCRPC patient to generate CTC data, wherein the analysis comprises detecting the presence of AR-V7 localized in the nucleus of CTCs, wherein the nuclear localization of the AR-V7 corresponds to a positive response to taxane therapy compared to ARS-directed therapy for the mCRPC patient.

In some embodiments, the nuclear localization comprises a staining pattern with signal intensity ≥3-fold higher than background staining from neighboring white blood cells (WBCs).

### BRIEF DESCRIPTION OF THE DRAWINGS

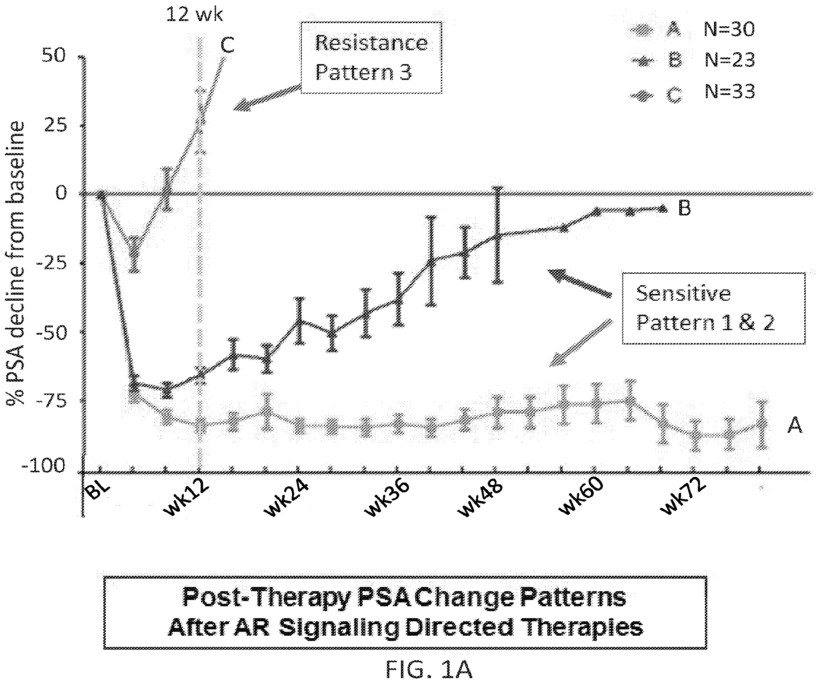

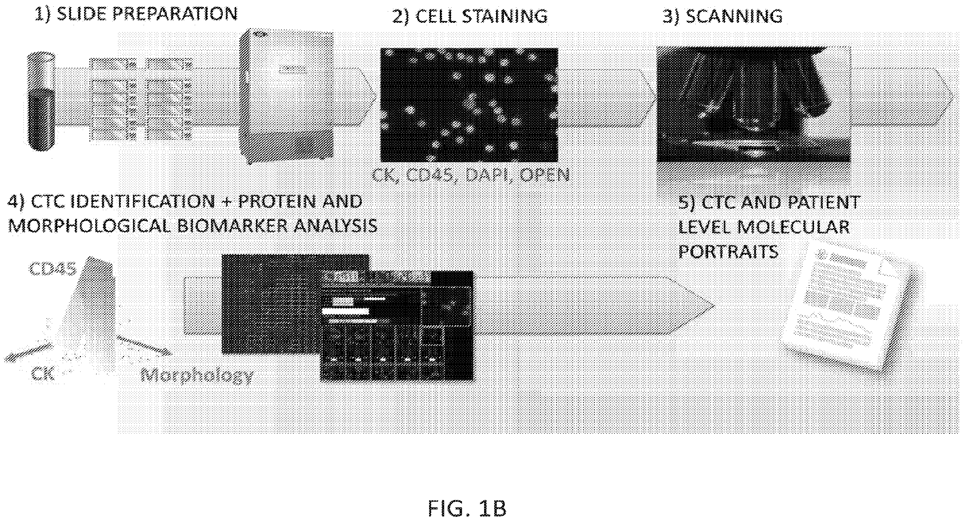

Figure 1A, shows post-therapy PSA change patterns after AR signaling directed therapies. 193 mCRPC patient blood samples were collected prior to starting Abiraterone (44); Enzalutamide (81), Docetaxel (46) Cabazitaxel (13), and Paclitaxel (2). PSA Outcomes were recorded as Sensitive (S): Patterns 1 and 2, or Resistant (R): Pattern 3 (A). Scher et al. Cancer J. 2013 Jan-Feb;19(1):43-9. Patients were monitored for up to 2.3 yrs to assess rPFS and OS outcomes. Samples were processed utilizing the Epic Sciences platform for CTC enumeration, morphology, biomarker, & FISH analyses workflow shown schematically in Figure 1B: 1) Nucleated cells from blood sample placed onto slides and stored in a -80∘C biorepository; 2) Slides stained with cytokeratin (CK), CD45, DAPI, AR-V7; 3) Slides scanned; 4) CTC candidates detected by a multi-parametric digital pathology algorithm, and 5) Human reader confirmation of CTCs & quantitation of biomarker expression.

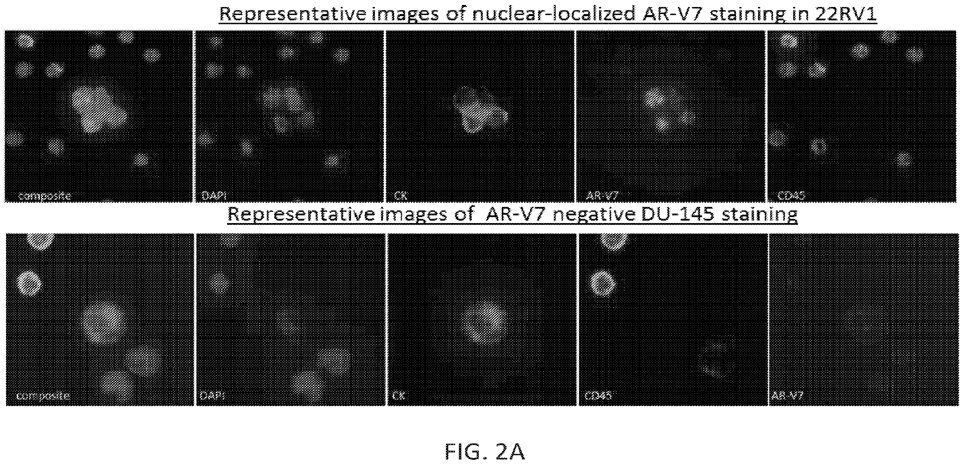

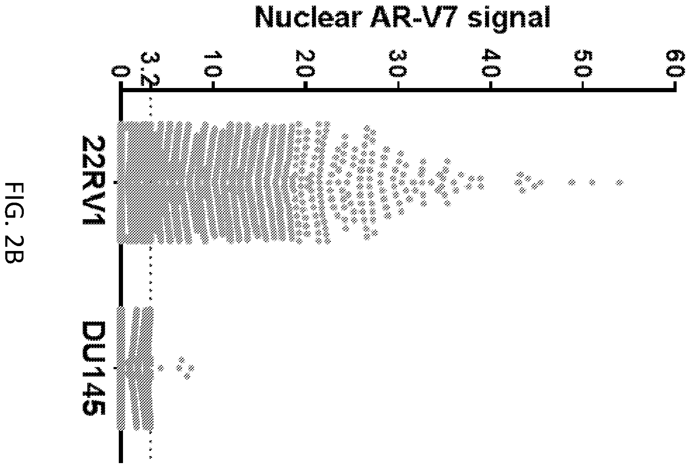

Figures 2A and 2B show AR-V7 IF assay development. A rabbit monoclonal antibody specific to the AR-V7 splice variant was used for IF staining of cell line controls and patient CTCs characterized by the Epic CTC Platform. Positive (22RV1), Figure 2A, upper, and negative (DU145), Figure 2A, lower, cell line controls were used to assess initial AR-V7 IF protein assay. AR-V7 positive cells were defined as cells having a specific nuclear-localized staining pattern with signal intensity >3-fold higher than background staining from neighboring WBCs. Figure 2B depicts a graph showing that 55% (2660/4813) of individually characterized 22RV1 cells showed specific AR-V7 staining, while nuclear staining was seen in only 0.001% (5/3360) of DU145 negative controls.

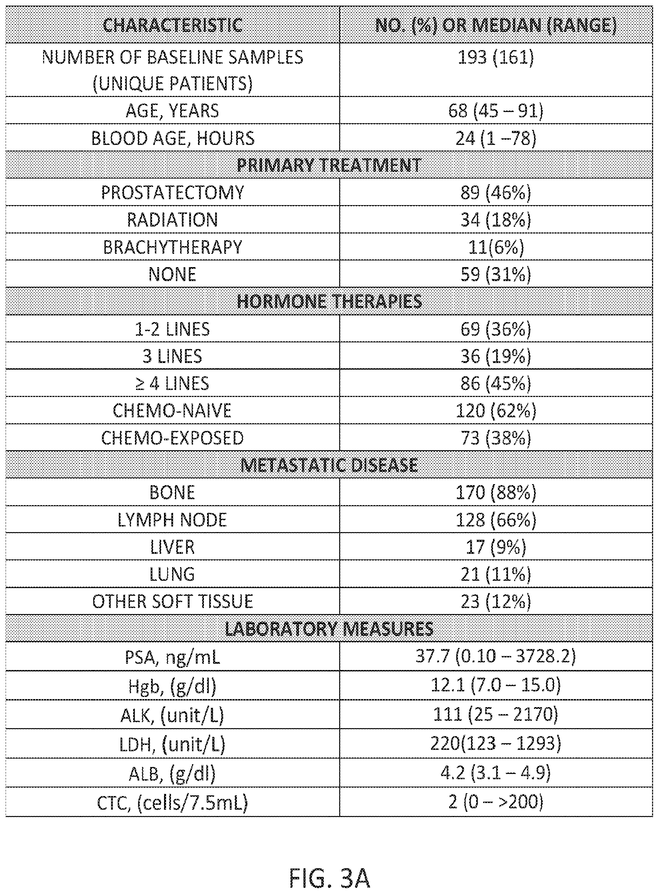

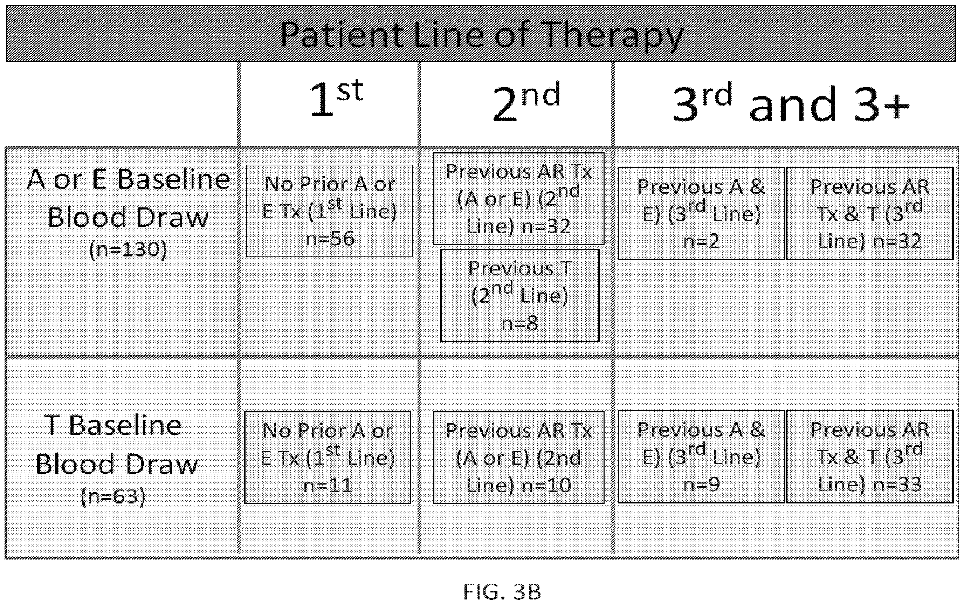

Figures 3A and 3B show patient demographics. Figure 3A shows patient characteristics, while Figure 3B shows patient line of therapy at the time the samples were obtained.

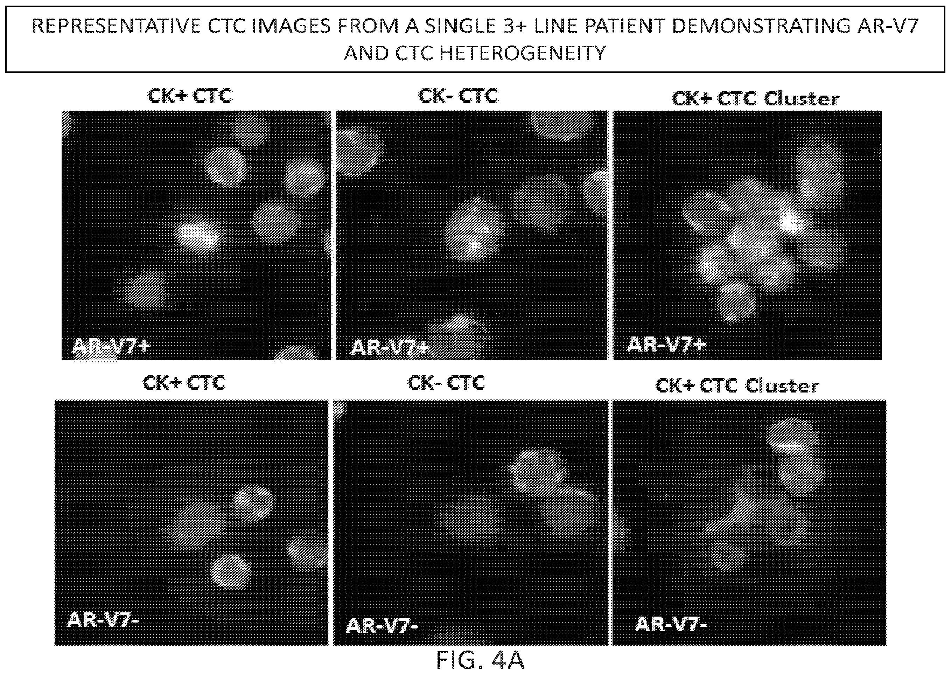

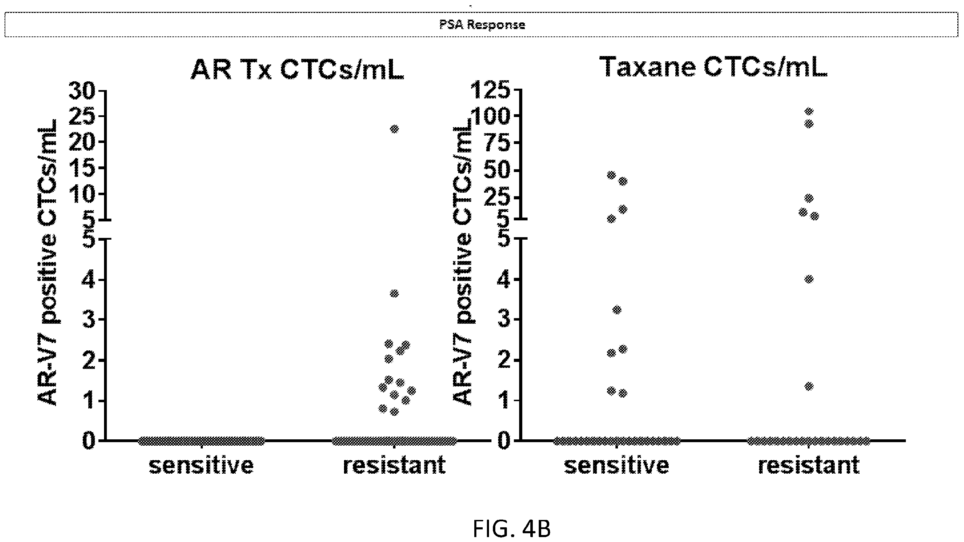

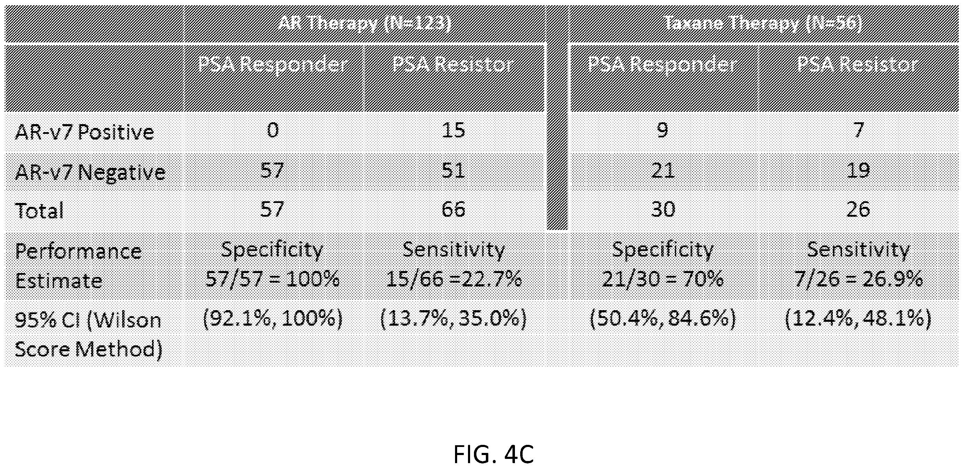

Figures 4A-4C show that baseline AR-V7+ CTCs predict PSA resistance to AR therapy but not to taxane chemotherapy. Figure 4A shows representative CTC images from a single 3+ Line patient demonstrating AR-V7 and CTC heterogeneity. Figure 4B shows graphs depicting the number of AR-V7 positive CTCs per ml in AR therapy resistant and sensitive patients (left) and the number of AR-V7 positive CTCs per ml in taxane therapy resistant and sensitive patients (right). Figure 4C shows a table demonstrating that AR-V7 positivity predicts PSA resistance to AR-directed therapy. Briefly, AR-V7 protein expression was found in CTCs from 15/66 patients resistant to AR Therapy. While CTCs were identified in 37/57 AR Therapy sensitive patients (range: is 0 to 345 CTCs/mL , median: 2 CTCs/mL), 0/57 harbored AR-V7+ CTCs. Of all patients in the AR Therapy cohort that lacked AR-V7+ cells, 53% were sensitive to AR Therapy. AR-V7 prevalence does not predict resistance to Taxane chemotherapy: AR-V7+ CTCs were found in 9/30 and 7/26 Taxane-sensitive and resistant patients, respectively.

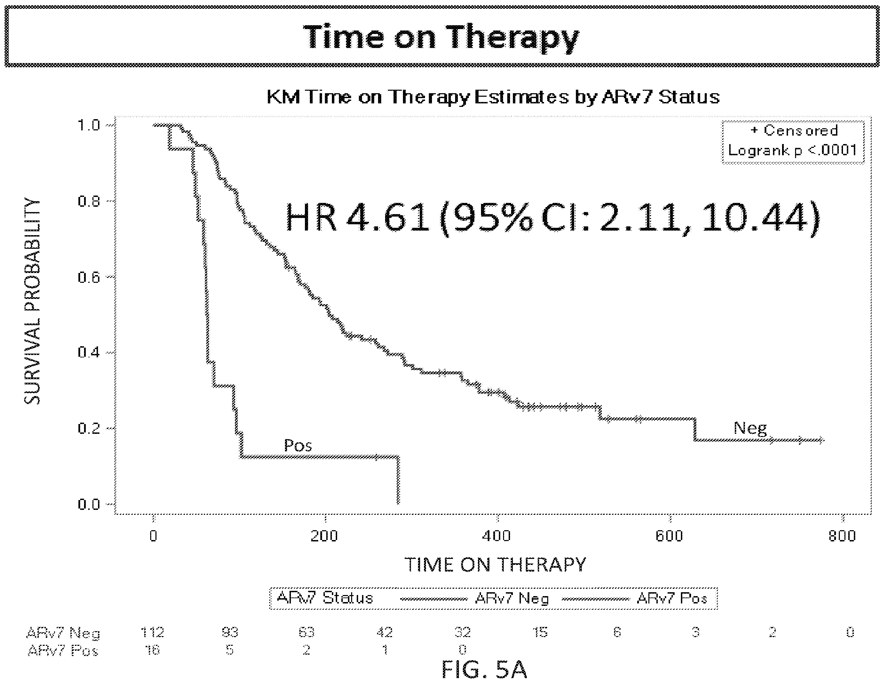

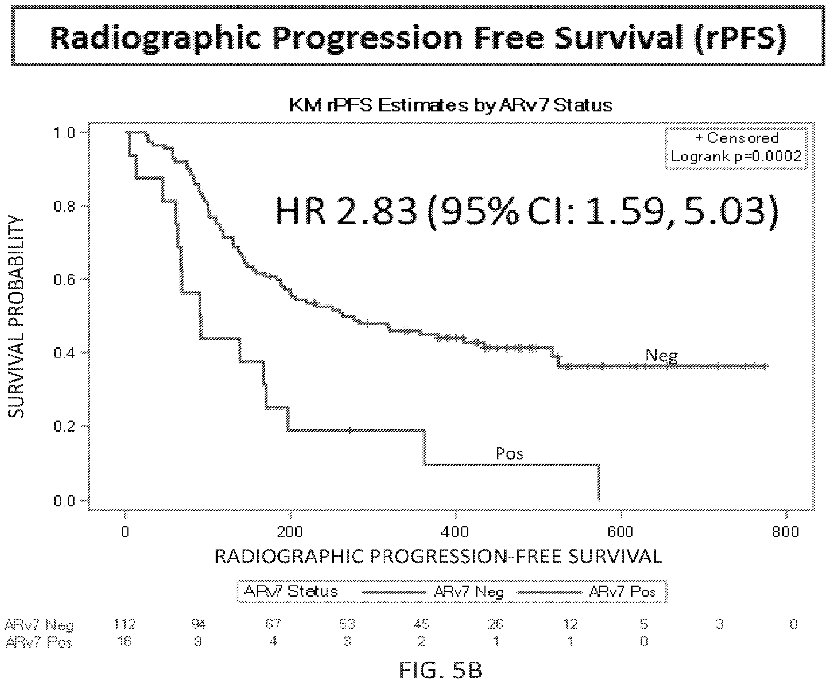

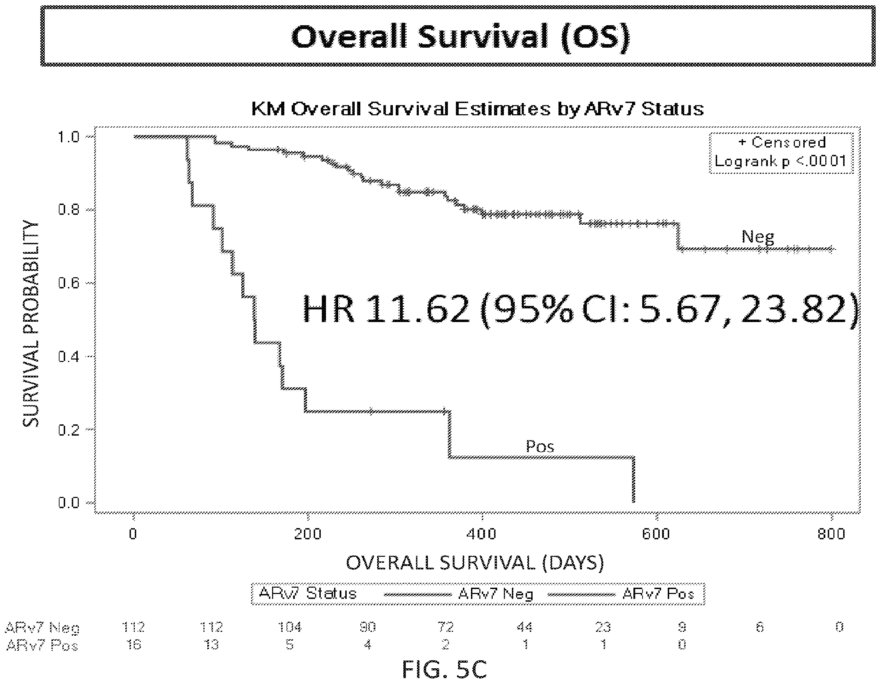

Figures 5A-5C demonstrate that baseline AR-V7+ CTCs predict unfavorable outcomes on AR therapy and that, in patients on AR-directed therapies, AR-V7 positive status is associated with shorter time on therapy (Figure 5A), shorter radiographic Progression Free Survival(rPFS) (Figure 5B) and shorter overall survival (OS) (Figure 5C).

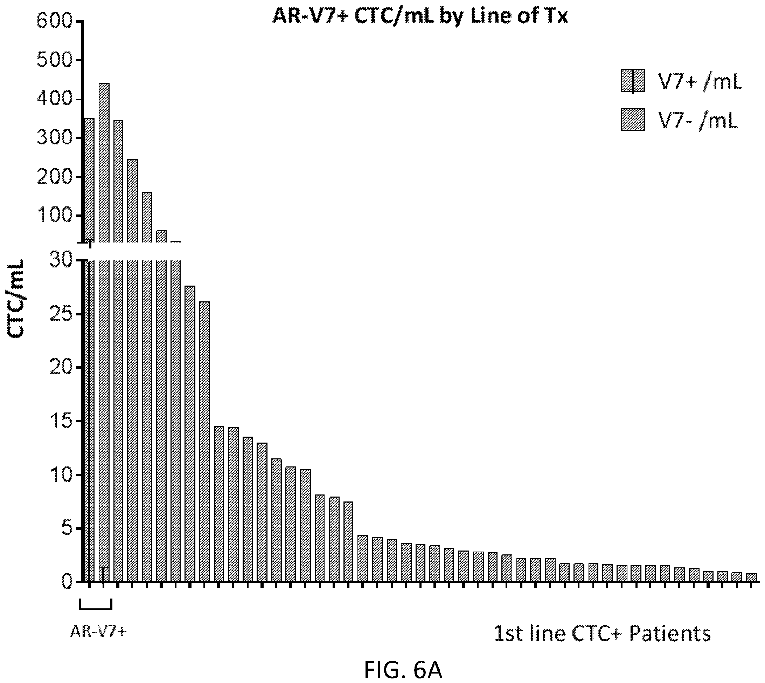

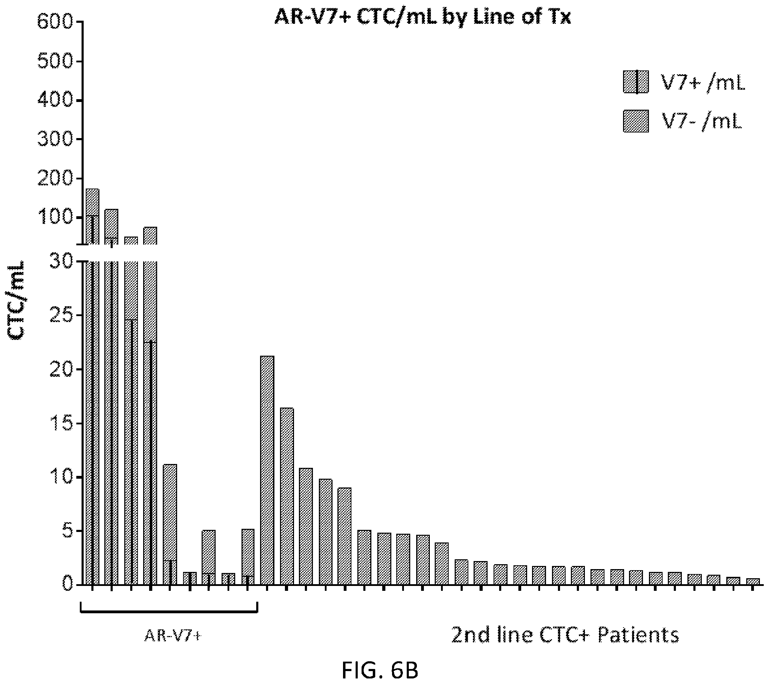

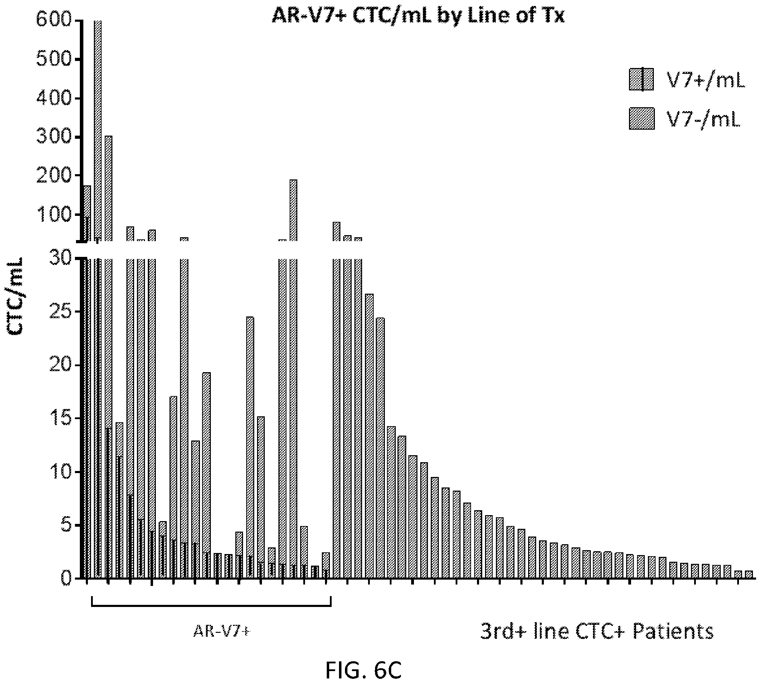

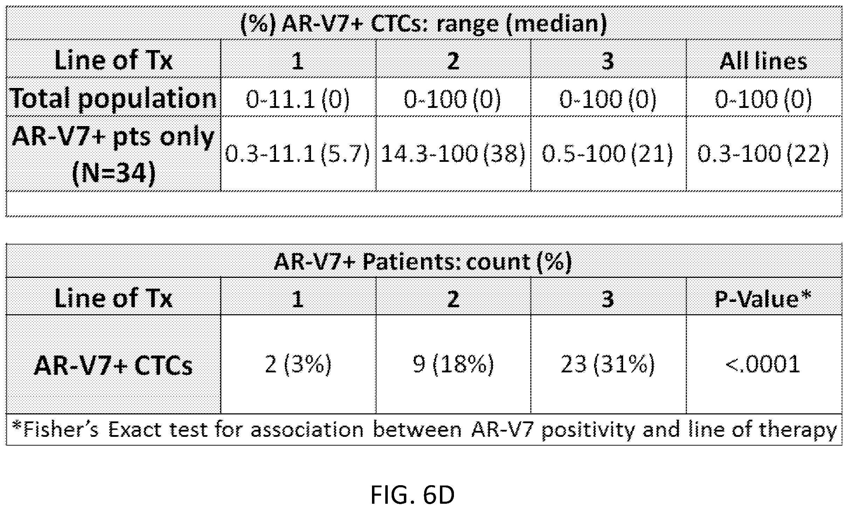

Figures 6A-6D show heterogeneity and prevalence of AR-V7+ CTCs, and specifically shows that frequency and heterogeneity of AR-V7 positivity increased in patients with increasing lines of therapy. CTCs were identified in 144/191 (75%) patients. AR-V7+ CTCs were found in 2/67 (3%) first line (Figure 6A) , 9/50 (18%) second line (Figure 6B), and 23/74 (31%) third or greater lines (Figure 6C). Figure 6D depicts a table showing that frequency and heterogeneity of AR-V7 positivity increased in patients with increasing lines of therapy.

### DETAILED DESCRIPTION

The present disclosure describes an AR-V7 immunofluorescent test for use in fixed single CTCs, inclusive of all CTC subtypes utilizing samples from progressive mCRPC patients in need of a change in therapy on a platform designed for global diagnostic workflows.

The present invention provides a method of identifying a metastatic castration resistant prostate cancer (mCRPC) patient with an improved response to taxane therapy compared to androgen receptor (AR) targeted therapy comprising (a) performing a direct analysis comprising immunofluorescent staining and morphological characterization of nucleated cells in a blood sample obtained from the patient to generate circulating tumor cell (CTC) data, wherein the direct analysis means the detection of the CTCs in the context of all surrounding nucleated cells present in the sample as opposed to after enrichment of the sample for CTCs prior to detection, and wherein the analysis comprises detecting the presence of an Androgen Receptor Variant 7 (AR-V7) in said cells, and (b) evaluating the CTC data to identify a mCRPC patient with an improved response to taxane therapy compared to ARS-directed therapy, wherein the presence of an Androgen Receptor Variant 7 in said CTCs identifies a mCRPC patient with an improved response to taxane therapy compared to ARS-directed therapy. .

As used herein, the term "circulating tumor cell" or "CTC" is meant to encompass any rare cell that is present in a biological sample and that is related to prostate cancer including traditional and non-traditional CTCs. CTCs, which can be present as single cells or in clusters of CTCs, are often epithelial cells shed from solid tumors found in very low concentrations in the circulation of patients. A traditional CTC refers to a single CTC that is cytokeratin positive, CD45 negative, contains a DAPI nucleus, and is morphologically distinct from surrounding white blood cells. A non-traditional CTC refers to a CTC that differs from a traditional CTC in at least one characteristic. Non-traditional CTCs include the five CTC subtypes shown in Figure 2, Panel B, including CTC clusters, CK negative CTCs that are positive at least one additional biomarker that allows classification as a CTC , small CTCs, nucleoli ⁺ CTCs and CK speckled CTCs. As used herein, the term "CTC cluster" means two or more CTCs with touching cell membranes.

In its broadest sense, a biological sample can be any sample that contains CTCs. A sample can comprise a bodily fluid such as blood; the soluble fraction of a cell preparation, or an aliquot of media in which cells were grown; a chromosome, an organelle, or membrane isolated or extracted from a cell; genomic DNA, RNA, or cDNA in solution or bound to a substrate; a cell; a tissue; a tissue print; a fingerprint; cells; skin, and the like. A biological sample obtained from a subject can be any sample that contains cells and encompasses any material in which CTCs can be detected. A sample can be, for example, whole blood, plasma, saliva or other bodily fluid or tissue that contains cells.

In the invention, the biological sample is a blood sample. As described herein, a sample can be whole blood, more preferably peripheral blood or a peripheral blood cell fraction. As will be appreciated by those skilled in the art, a blood sample can include any fraction or component of blood, without limitation, T-cells, monocytes, neutrophiles, erythrocytes, platelets and microvesicles such as exosomes and exosome-like vesicles. In the context of this disclosure, blood cells included in a blood sample encompass any nucleated cells and are not limited to components of whole blood. As such, blood cells include, for example, both white blood cells (WBCs) as well as rare cells, including CTCs.

The samples of this disclosure can each contain a plurality of cell populations and cell subpopulation that are distinguishable by methods well known in the art (*e.g.*, FACS, immunohistochemistry). For example, a blood sample can contain populations of nonnucleated cells, such as erythrocytes (*e.g.*, 4-5 million/µl) or platelets (150,000-400,000 cells/µl), and populations of nucleated cells such as WBCs (*e.g.*, 4,500 ― 10,000 cells/µl), CECs or CTCs (circulating tumor cells; *e.g.*, 2-800 cells/). WBCs may contain cellular subpopulations of, *e.g.*, neutrophils (2,500-8,000 cells/µl), lymphocytes (1,000-4,000 cells/µl), monocytes (100-700 cells/µl), eosinophils (50-500 cells/µl), basophils (25 ― 100 cells/ µl) and the like. The samples of this disclosure are non-enriched samples, *i.e.*, they are not enriched for any specific population or subpopulation of nucleated cells. For example, non-enriched blood samples are not enriched for CTCs, WBC, B-cells, T-cells, NK-cells, monocytes, or the like.

The methods can be performed by methods known to those skilled in the art, for example, as described by Marrinucci et al. Hum Pathol 38(3): 514―519 (2007); Marrinucci et al. Arch Pathol Lab Med 133(9): 1468―1471 (2009); Mikolajczyk et al. J Oncol 2011: 252361. (2011); Marrinucci et al. Phys Biol 9(1): 016003 (2012); Werner et al. J Circ Biomark 4: 3 (2015).

As used herein in the context of generating CTC data, the term "direct analysis" means that the CTCs are detected in the context of all surrounding nucleated cells present in the sample as opposed to after enrichment of the sample for CTCs prior to detection. In some embodiments, the methods comprise microscopy providing a field of view that includes both CTCs and at least 200 surrounding white blood cells (WBCs).

The present disclosure also provides a method of performing a direct analysis comprising immunofluorescent staining and morphological characterization of nucleated cells in a blood sample obtained from a metastatic castration resistant prostate cancer (mCRPC) patient to generate circulating tumor cell (CTC) data, wherein the analysis comprises detecting the presence of an Androgen Receptor Variant 7 (AR-V7) in said cells.

The present disclosure also provides a method of performing a direct analysis comprising immunofluorescent staining and morphological characterization of nucleated cells in a blood sample obtained from a mCRPC patient to generate CTC data, wherein the analysis comprises detecting the presence of AR-V7 localized in the nucleus of CTCs.

The present disclosure also provides a method of performing a direct analysis comprising immunofluorescent staining and morphological characterization of nucleated cells in a blood sample obtained from a mCRPC patient to generate CTC data, wherein the analysis comprises detecting the presence of AR-V7 localized in the nucleus of CTCs, wherein the nuclear localization of the AR-V7 corresponds to resistance to ARS-directed therapy for the mCRPC patient.

The present disclosure also provides a method of performing a direct analysis comprising immunofluorescent staining and morphological characterization of nucleated cells in a blood sample obtained from a mCRPC patient to generate CTC data, wherein the analysis comprises detecting the presence of AR-V7 localized in the nucleus of CTCs, wherein the nuclear localization of the AR-V7 corresponds to a positive response to taxane therapy compared to ARS-directed therapy for the mCRPC patient.

In some embodiments of the methods described herein, the nuclear localization of AR-V7 comprises a staining pattern with signal intensity about ≥2-fold, ≥3-fold, ≥4-fold, ≥5-fold, ≥6-fold, ≥7-fold, ≥8-fold, ≥9-fold, ≥10-fold, ≥11-fold, ≥12-fold, ≥13-fold, ≥14-fold, ≥15-fold, ≥20-fold, ≥25-fold, ≥30-fold, or ≥35-fold higher than background staining from neighboring white blood cells (WBCs).

A fundamental aspect of the present disclosure is the unparalleled robustness of the disclosed methods with regard to the detection of CTCs. The rare event detection disclosed herein with regard to CTCs is based on a direct analysis, *i.e.* non-enriched, of a population that encompasses the identification of rare events in the context of the surrounding non-rare events. Identification of the rare events according to the disclosed methods inherently identifies the surrounding events as non-rare events. Taking into account the surrounding non-rare events and determining the averages for non-rare events, for example, average cell size of non-rare events, allows for calibration of the detection method by removing noise. The result is a robustness of the disclosed methods that cannot be achieved with methods that are not based on direct analysis, but that instead compare enriched populations with inherently distorted contextual comparisons of rare events. The robustness of the direct analysis methods disclosed herein enables characterization of CTC, including subtypes of CTCs described herein, that allows for identification of phenotypes and heterogeneity that cannot be achieved with other CTC detection methods and that enables the analysis of biomarkers in the context of the claimed methods.

CTC data can include both morphological features and immunofluorescent features. As will be understood by those skilled in the art, biomarkers can include a biological molecule, or a fragment of a biological molecule, the change and/or the detection of which can be correlated, individually or combined with other measurable features, with prostate cancer and/or mCRPC. CTCs, which can be present a single cells or in clusters of CTCs, are often epithelial cells shed from solid tumors and are present in very low concentrations in the circulation of subjects. Accordingly, detection of CTCs in a blood sample can be referred to as rare event detection. CTCs have an abundance of less than 1:1,000 in a blood cell population, *e.g*., an abundance of less than 1:5,000, 1:10,000, 1:30,000, 1:50:000, 1:100,000, 1:300,000, 1:500,000, or 1:1,000,000. In some embodiments, the a CTC has an abundance of 1:50:000 to 1:100,000 in the cell population.

The samples of this disclosure may be obtained by any means, including, *e.g*., by means of solid tissue biopsy or fluid biopsy *(see, e.g.,* Marrinucci D. et al., 2012, Phys. Biol. 9 016003). Briefly, in particular embodiments, the process can encompass lysis and removal of the red blood cells in a 7.5 mL blood sample, deposition of the remaining nucleated cells on specialized microscope slides, each of which accommodates the equivalent of roughly 0.5 mL of whole blood. A blood sample may be extracted from any source known to include blood cells or components thereof, such as venous, arterial, peripheral, tissue, cord, and the like. The samples may be processed using well known and routine clinical methods (*e*.*g*., procedures for drawing and processing whole blood). In some embodiments, a blood sample is drawn into anti-coagulent blood collection tubes (BCT), which may contain EDTA or Streck Cell-Free DNA™. In other embodiments, a blood sample is drawn into CellSave® tubes (Veridex). A blood sample may further be stored for up to 12 hours, 24 hours, 36 hours, 48 hours, or 60 hours before further processing.

In some embodiments, the methods of this disclosure comprise an intitial step of obtaining a white blood cell (WBC) count for the blood sample. In certain embodiments, the WBC count may be obtained by using a HemoCue® WBC device (Hemocue, Ängelholm, Sweden). In some embodiments, the WBC count is used to determine the amount of blood required to plate a consistent loading volume of nucleated cells per slide and to calculate back the equivalent of CTCs per blood volume.

In some embodiments, the methods of this disclosure comprise an initial step of lysing erythrocytes in the blood sample. In some embodiments, the erythrocytes are lysed, *e.g.,* by adding an ammonium chloride solution to the blood sample. In certain embodiments, a blood sample is subjected to centrifugation following erythrocyte lysis and nucleated cells are resuspended, *e*.*g*., in a PBS solution.

In some embodiments, nucleated cells from a sample, such as a blood sample, are deposited as a monolayer on a planar support. The planar support may be of any material, *e.g.,* any fluorescently clear material, any material conducive to cell attachment, any material conducive to the easy removal of cell debris, any material having a thickness of < 100 µm. In some embodiments, the material is a film. In some embodiments the material is a glass slide. In certain embodiments, the method encompasses an initial step of depositing nucleated cells from the blood sample as a monolayer on a glass slide. The glass slide can be coated to allow maximal retention of live cells *(See, e.g.,* Marrinucci D. et al., 2012, Phys. Biol. 9 016003). In some embodiments, about 0.5 million, 1 million, 1.5 million, 2 million, 2.5 million, 3 million, 3.5 million, 4 million, 4.5 million, or 5 million nucleated cells are deposited onto the glass slide. In some embodiments, the methods of this disclosure comprise depositing about 3 million cells onto a glass slide. In additional embodiments, the methods of this disclosure comprise depositing between about 2 million and about 3 million cells onto the glass slide. In some embodiments, the glass slide and immobilized cellular samples are available for further processing or experimentation after the methods of this disclosure have been completed.

In some embodiments, the methods of this disclosure comprise an initial step of identifying nucleated cells in the non-enriched blood sample. In some embodiments, the nucleated cells are identified with a fluorescent stain. In certain embodiments, the fluorescent stain comprises a nucleic acid specific stain. In certain embodiments, the fluorescent stain is diamidino-2-phenylindole (DAPI). In some embodiments, immunofluorescent staining of nucleated cells comprises pan cytokeratin (CK), cluster of differentiation (CD) 45 and DAPI. In some embodiments further described herein, CTCs comprise distinct immunofluorescent staining from surrounding nucleated cells. In some embodiments, the distinct immunofluorescent staining of CTCs comprises DAPI (+), CK (+) and CD 45 (-). In some embbodiments, the identification of CTCs further comprises comparing the intensity of pan cytokeratin fluorescent staining to surrounding nucleated cells. In some embodiments, the CTC data is generated by fluorescent scanning microscopy to detect immunofluorescent staining of nucleated cells in a blood sample. Marrinucci D. et al., 2012, Phys. Biol. 9 016003).

In particular embodiments, all nucleated cells are retained and immunofluorescently stained with monoclonal antibodies targeting cytokeratin (CK), an intermediate filament found exclusively in epithelial cells, a pan leukocyte specific antibody targeting the common leukocyte antigen CD45, and a nuclear stain, DAPI. The nucleated blood cells can be imaged in multiple fluorescent channels to produce high quality and high resolution digital images that retain fine cytologic details of nuclear contour and cytoplasmic distribution. While the surrounding WBCs can be identified with the pan leukocyte specific antibody targeting CD45, CTCs can be identified as DAPI (+), CK (+) and CD 45 (-). In the methods described herein, the CTCs comprise distinct immunofluorescent staining from surrounding nucleated cells.

In further embodiments, the CTC data includes traditional CTCs also known as high definition CTCs (HD-CTCs) . Traditional CTCs are CK positive, CD45 negative, contain an intact DAPI positive nucleus without identifiable apoptotic changes or a disrupted appearance, and are morphologically distinct from surrounding white blood cells (WBCs). DAPI (+), CK (+) and CD45 (-) intensities can be categorized as measurable features during HD-CTC enumeration as previously described. Nieva et al., Phys Biol 9:016004 (2012). The enrichment-free, direct analysis employed by the methods disclosed herein results in high sensitivity and high specificity, while adding high definition cytomorphology to enable detailed morphologic characterization of a CTC population known to be heterogeneous.

While CTCs can be identified as comprises DAPI (+), CK (+) and CD 45 (-) cells, the methods of the invention can be practiced with any other biomarkers that one of skill in the art selects for generating CTC data and/or identifying CTCs and CTC clusters. One skilled in the art knows how to select a morphological feature, biological molecule, or a fragment of a biological molecule, the change and/or the detection of which can be correlated with a CTC. Molecule biomarkers include, but are not limited to, biological molecules comprising nucleotides, nucleic acids, nucleosides, amino acids, sugars, fatty acids, steroids, metabolites, peptides, polypeptides, proteins, carbohydrates, lipids, hormones, antibodies, regions of interest that serve as surrogates for biological macromolecules and combinations thereof (e.g., glycoproteins, ribonucleoproteins, lipoproteins). The term also encompasses portions or fragments of a biological molecule, for example, peptide fragment of a protein or polypeptide

A person skilled in the art will appreciate that a number of methods can be used to generate CTC data, including microscopy based approaches, including fluorescence scanning microscopy *(see, e.g.,* Marrinucci D. et al., 2012, Phys. Biol. 9 016003), mass spectrometry approaches, such as MS/MS, LC-MS/MS, multiple reaction monitoring (MRM) or SRM and product-ion monitoring (PIM) and also including antibody based methods such as immunofluorescence, immunohistochemistry, immunoassays such as Western blots, enzymelinked immunosorbant assay (ELISA), immunopercipitation, radioimmunoassay, dot blotting, and FACS. Immunoassay techniques and protocols are generally known to those skilled in the art (Price and Newman, Principles and Practice of Immunoassay, 2nd Edition, Grove's Dictionaries, 1997; and Gosling, Immunoassays: A Practical Approach, Oxford University Press, 2000.) A variety of immunoassay techniques, including competitive and noncompetitive immunoassays, can be used (Self et al., Curr. Opin. Biotechnol., 7:60-65 (1996), *see also* John R. Crowther, The ELISA Guidebook, 1st ed., Humana Press 2000, ISBN 0896037282 and, An Introduction to Radioimmunoassay and Related Techniques, by Chard T, ed., Elsevier Science 1995, ISBN 0444821198).

A person of skill in the art will futher appreciate that the presence or absence of biomarkers may be detected using any class of marker-specific binding reagents known in the art, including, *e.g*., antibodies, aptamers, fusion proteins, such as fusion proteins including protein receptor or protein ligand components, or biomarker-specific small molecule binders. In some embodiments, the presence or absence of CK, AR-V7 or CD45 is determined by an antibody.

The antibodies of this disclosure bind specifically to a biomarker. The antibody can be prepared using any suitable methods known in the art. *See, e.g.,* Coligan, Current Protocols in Immunology (1991); Harlow & Lane, Antibodies: A Laboratory Manual (1988); Goding, Monoclonal Antibodies: Principles and Practice (2d ed. 1986). The antibody can be any immunoglobulin or derivative thereof, whether natural or wholly or partially synthetically produced. All derivatives thereof which maintain specific binding ability are also included in the term. The antibody has a binding domain that is homologous or largely homologous to an immunoglobulin binding domain and can be derived from natural sources, or partly or wholly synthetically produced. The antibody can be a monoclonal or polyclonal antibody. In some embodiments, an antibody is a single chain antibody. Those of ordinary skill in the art will appreciate that antibody can be provided in any of a variety of forms including, for example, humanized, partially humanized, chimeric, chimeric humanized, *etc.* The antibody can be an antibody fragment including, but not limited to, Fab, Fab', F(ab')2, scFv, Fv, dsFv diabody, and Fd fragments. The antibody can be produced by any means. For example, the antibody can be enzymatically or chemically produced by fragmentation of an intact antibody and/or it can be recombinantly produced from a gene encoding the partial antibody sequence. The antibody can comprise a single chain antibody fragment. Alternatively or additionally, the antibody can comprise multiple chains which are linked together, for example, by disulfide linkages, and any functional fragments obtained from such molecules, wherein such fragments retain specific-binding properties of the parent antibody molecule. Because of their smaller size as functional components of the whole molecule, antibody fragments can offer advantages over intact antibodies for use in certain immunochemical techniques and experimental applications.

A detectable label can be used in the methods described herein for direct or indirect detection of the biomarkers when generating CTC data in the methods of the invention. A wide variety of detectable labels can be used, with the choice of label depending on the sensitivity required, ease of conjugation with the antibody, stability requirements, and available instrumentation and disposal provisions. Those skilled in the art are familiar with selection of a suitable detectable label based on the assay detection of the biomarkers used in in the methods of the invention. Suitable detectable labels include, but are not limited to, fluorescent dyes (*e.g.*, fluorescein, fluorescein isothiocyanate (FITC), Oregon Green™, rhodamine, Texas red, tetrarhodimine isothiocynate (TRITC), Cy3, Cy5, Alexa Fluor® 647, Alexa Fluor® 555, Alexa Fluor® 488), fluorescent markers (*e.g*., green fluorescent protein (GFP), phycoerythrin, etc.), enzymes (*e.g.*, luciferase, horseradish peroxidase, alkaline phosphatase, etc.), nanoparticles, biotin, digoxigenin, metals, and the like.

For mass-sectrometry based analysis, differential tagging with isotopic reagents, *e.g.*, isotope-coded affinity tags (ICAT) or the more recent variation that uses isobaric tagging reagents, iTRAQ (Applied Biosystems, Foster City, Calif.), followed by multidimensional liquid chromatography (LC) and tandem mass spectrometry (MS/MS) analysis can provide a further methodology in practicing the methods of this disclosure.

A chemiluminescence assay using a chemiluminescent antibody can be used for sensitive, non-radioactive detection of proteins. An antibody labeled with fluorochrome also can be suitable. Examples of fluorochromes include, without limitation, DAPI, fluorescein, Hoechst 33258, R-phycocyanin, B-phycoerythrin, R-phycoerythrin, rhodamine, Texas red, and lissamine. Indirect labels include various enzymes well known in the art, such as horseradish peroxidase (HRP), alkaline phosphatase (AP), beta-galactosidase, urease, and the like. Detection systems using suitable substrates for horseradish-peroxidase, alkaline phosphatase, beta.-galactosidase are well known in the art.

A signal from the direct or indirect label can be analyzed, for example, using a microscope, such as a fluorescence microscope or a fluorescence scanning microscope. Alternatively, a spectrophotometer can be used to detect color from a chromogenic substrate; a radiation counter to detect radiation such as a gamma counter for detection of ¹²⁵I; or a fluorometer to detect fluorescence in the presence of light of a certain wavelength. If desired, assays used to practice the methods of this disclosure can be automated or performed robotically, and the signal from multiple samples can be detected simultaneously.

In some embodiments, the biomarkers are immunofluorescent markers. In some embodiments, the immunofluorescent makers comprise a marker specific for epithelial cells In some embodiments, the immunofluorescent makers comprise a marker specific for white blood cells (WBCs). In some embodiments, one or more of the immunofluorescent markers comprise CD 45 and CK.

In some embodiments, the presence or absence of immunofluorescent markers in nucleated cells, such as CTCs or WBCs, results in distinct immunofluorescent staining patterns. Immunofluorescent staining patterns for CTCs and WBCs may differ based on which epithelial or WBC markers are detected in the respective cells. In some embodiments, determining presence or absence of one or more immunofluorescent markers comprises comparing the distinct immunofluorescent staining of CTCs with the distinct immunofluorescent staining of WBCs using, for example, immunofluorescent staining of CD45, which distinctly identifies WBCs. There are other detectable markers or combinations of detectable markers that bind to the various subpopulations of WBCs. These may be used in various combinations, including in combination with or as an alternative to immunofluorescent staining of CD45.

In some embodiments, CTCs comprise distinct morphological characteristics compared to surrounding nucleated cells. In some embodiments, the morphological characteristics comprise nucleus size, nucleus shape, cell size, cell shape, and/or nuclear to cytoplasmic ratio. In some embodiments, the method further comprises analyzing the nucleated cells by nuclear detail, nuclear contour, presence or absence of nucleoli, quality of cytoplasm, quantity of cytoplasm, intensity of immunofluorescent staining patterns. A person of ordinary skill in the art understands that the morphological characteristics of this disclosure may include any feature, property, characteristic, or aspect of a cell that can be determined and correlated with the detection of a CTC.

CTC data can be generated with any microscopic method known in the art. In some embodiments, the method is performed by fluorescent scanning microscopy. In certain embodiments the microscopic method provides high-resolution images of CTCs and their surrounding WBCs *(see, e.g.,* Marrinucci D. et al., 2012, Phys. Biol. 9 016003)). In some embodiments, a slide coated with a monolayer of nucleated cells from a sample, such as a non-enriched blood sample, is scanned by a fluorescent scanning microscope and the fluorescence intensities from immunofluorescent markers and nuclear stains are recorded to allow for the determination of the presence or absence of each immunofluorescent marker and the assessment of the morphology of the nucleated cells. In some embodiments, microscopic data collection and analysis is conducted in an automated manner.

In some embodiments, a CTC data includes detecting one or more biomarkers, for example, CK, AR-V7 and CD 45. A biomarker is considered "present" in a cell if it is detectable above the background noise of the respective detection method used (*e.g.*, 2-fold, 3-fold, 5-fold, or 10-fold higher than the background; *e.g.*, 2σ or 3σ over background). In some embodiments, a biomarker is considered "absent" if it is not detectable above the background noise of the detection method used (*e.g.*, <1.5-fold or <2.0-fold higher than the background signal; *e.g.*, <1.5σ or <2.0σ over background).

In some embodiments, the presence or absence of immunofluorescent markers in nucleated cells is determined by selecting the exposure times during the fluorescence scanning process such that all immunofluorescent markers achieve a pre-set level of fluorescence on the WBCs in the field of view. Under these conditions, CTC-specific immunofluorescent markers, even though absent on WBCs are visible in the WBCs as background signals with fixed heights. Moreover, WBC-specific immunofluorescent markers that are absent on CTCs are visible in the CTCs as background signals with fixed heights. A cell is considered positive for an immunofluorescent marker (*i.e.*, the marker is considered present) if its fluorescent signal for the respective marker is significantly higher than the fixed background signal (*e.g.*, 2-fold, 3-fold, 5-fold, or 10-fold higher than the background; *e.g.*, 2σ or 3σ over background). For example, a nucleated cell is considered CD 45 positive (CD 45⁺) if its fluorescent signal for CD 45 is significantly higher than the background signal. A cell is considered negative for an immunofluorescent marker (*i.e.*, the marker is considered absent) if the cell's fluorescence signal for the respective marker is not significantly above the background signal (*e.g.*, <1.5-fold or <2.0-fold higher than the background signal; *e.g.*, <1.5σ or <2.0σ over background).

Typically, each microscopic field contains both CTCs and WBCs. In certain embodiments, the microscopic field shows at least 1, 5, 10, 20, 50, or 100 CTCs. In certain embodiments, the microscopic field shows at least 10, 25, 50, 100, 250, 500, or 1,000 fold more WBCs than CTCs. In certain embodiments, the microscopic field comprises one or more CTCs or CTC clusters surrounded by at least 10, 50, 100, 150, 200, 250, 500, 1,000 or more WBCs.

In some embodiments of the methods described herein, generation of the CTC data comprises enumeration of CTCs that are present in the blood sample. In some embodiments, the methods described herein encompass detection of at least 1.0 CTC/mL of blood, 1.5 CTCs/mL of blood, 2.0 CTCs/mL of blood, 2.5 CTCs/mL of blood, 3.0 CTCs/mL of blood, 3.5 CTCs/mL of blood, 4.0 CTCs/mL of blood, 4.5 CTCs/mL of blood, 5.0 CTCs/mL of blood, 5.5 CTCs/mL of blood, 6.0 CTCs/mL of blood, 6.5 CTCs/mL of blood, 7.0 CTCs/mL of blood, 7.5 CTCs/mL of blood, 8.0 CTCs/mL of blood, 8.5 CTCs/mL of blood, 9.0 CTCs/mL of blood, 9.5 CTCs/mL of blood, 10 CTCs/mL of blood, or more.

In some embodiments of methods described herein, generation of the CTC data comprises detecting distinct subtypes of CTCs, including non-traditional CTCs. In some embodiments, the methods described herein encompass detection of at least 0.1 CTC cluster/mL of blood, 0.2 CTC clusters/mL of blood, 0.3 CTC clusters/mL of blood, 0.4 CTC clusters/mL of blood, 0.5 CTC clusters/mL of blood, 0.6 CTC clusters/mL of blood, 0.7 CTC clusters/mL of blood, 0.8 CTC clusters/mL of blood, 0.9 CTC clusters/mL of blood, 1 CTC cluster/mL of blood, 2 CTC clusters/mL of blood, 3 CTC clusters/mL of blood, 4 CTC clusters/mL of blood, 5 CTC clusters/mL of blood, 6 CTC clusters/mL of blood, 7 CTC clusters/mL of blood, 8 CTC clusters/mL of blood, 9 CTC clusters/mL of blood, 10 clusters/mL or more. In a particular embodiment, the methods described herein encompass detection of at least 1 CTC cluster/mL of blood.

In some embodiments, the methods comprise genomic analysis of the CTCs, for example, by fluorescence in situ hybridization (FISH).

Standard molecular biology techniques known in the art and not specifically described are generally followed as in Sambrook et al., Molecular Cloning: A Laboratory Manual, Cold Spring Harbor Laboratory Press, New York (1989), and as in Ausubel et al., Current Protocols in Molecular Biology, John Wiley and Sons, Baltimore, Md. (1989) and as in Perbal, A Practical Guide to Molecular Cloning, John Wiley & Sons, New York (1988), and as in Watson et al., Recombinant DNA, Scientific American Books, New York and in Birren et al (eds) Genome Analysis: A Laboratory Manual Series, Vols. 1-4 Cold Spring Harbor Laboratory Press, New York (1998). Polymerase chain reaction (PCR) can be carried out generally as in PCR Protocols: A Guide to Methods and Applications, Academic Press, San Diego, Calif. (1990). Any method capable of determining a DNA copy number profile of a particular sample can be used for molecular profiling provided the resolution is sufficient to identify the biomarkers used in the invention. The skilled artisan is aware of and capable of using a number of different platforms for assessing whole genome copy number changes at a resolution sufficient to identify the copy number of the one or more biomarkers used in the invention.

*In situ* hybridization assays are well known and are generally described in Angerer et al., Methods Enzymol. 152:649-660 (1987). In an in situ hybridization assay, cells, e.g., from a biopsy, are fixed to a solid support, typically a glass slide. If DNA is to be probed, the cells are denatured with heat or alkali. The cells are then contacted with a hybridization solution at a moderate temperature to permit annealing of specific probes that are labeled. The probes are preferably labeled with radioisotopes or fluorescent reporters. FISH (fluorescence in situ hybridization) uses fluorescent probes that bind to only those parts of a sequence with which they show a high degree of sequence similarity.

FISH is a cytogenetic technique used to detect and localize specific polynucleotide sequences in cells. For example, FISH can be used to detect DNA sequences on chromosomes. FISH can also be used to detect and localize specific RNAs, e.g., mRNAs, within tissue samples. In FISH uses fluorescent probes that bind to specific nucleotide sequences to which they show a high degree of sequence similarity. Fluorescence microscopy can be used to find out whether and where the fluorescent probes are bound. In addition to detecting specific nucleotide sequences, e.g., translocations, fusion, breaks, duplications and other chromosomal abnormalities, FISH can help define the spatial-temporal patterns of specific gene copy number and/or gene expression within cells and tissues.

The following example is provided by way of illustration, not limitation.

### EXAMPLE

### Example 1. Presence of AR-V7 splice variant in the CTCs of mCRPC patients identifies patients with improved PSA response with taxane therapy over androgen receptor signaling directed therapies (ARS Tx)

This example demonstrates that the presence of AR-V7 splice variant in the CTCs of mCRPC patients identifies patients with improved PSA response with taxane therapy over androgen receptor signaling directed therapies (ARS Tx)

Briefly, peripheral blood sample was collected in Cell-free DNA BCT (Streck, Omaha, NE, USA) and shipped immediately to Epic Sciences (San Diego, CA, USA) at ambient temperature. Upon receipt, red blood cells were lysed and nucleated cells were dispensed onto glass microscope slides as previously described (Marrinucci et al. Hum Pathol 38(3): 514―519 (2007); Marrinucci et al. Arch Pathol Lab Med 133(9): 1468―1471 (2009); Mikolajczyk et al. J Oncol 2011: 252361. (2011); Marrinucci et al. Phys Biol 9(1): 016003 (2012); Werner et al. J Circ Biomark 4: 3 (2015)) and stored at ―80 °C until staining. The millilitre equivalent of blood plated per slide was calculated based upon the sample's white blood cell count and the volume of post-RBC lysis cell suspension used. Circulating tumour cells were identified by immunofluorescence, as described (Marrinucci et al, 2007, *supra*; Marrinucci et al, 2009, *supra*; Mikolajczyk et al, 2011, *supra*; Marrinucci et al, 2012, *supra*; Werner et al, 2015, *supra*).

AR-V7 IF expression in CTCs of mCRPC patients assessed through the Epic Sciences platform is compatible with diagnostic workflows (median 24 hours from blood draw to processing). 193 mCRPC patient blood samples were collected prior to starting Abiraterone (44); Enzalutamide (81), Docetaxel (46) Cabazitaxel (13), and Paclitaxel (2). PSA Outcomes were recorded as Sensitive (S): Patterns 1 and 2, or Resistant (R): Pattern 3 (A) (Figure 1). Scher et al. Cancer J. 2013 Jan-Feb;19(1):43-9. Patients were monitored for up to 2.3 yrs to assess rPFS and OS outcomes. Samples were processed utilizing the Epic Sciences platform for CTC enumeration, morphology, biomarker, & FISH analyses workflow: 1) Nucleated cells from blood sample placed onto slides and stored in a -80∘C biorepository; 2) Slides stained with cytokeratin (CK), CD45, DAPI, AR-V7; 3) Slides scanned; 4) CTC candidates detected by a multi-parametric digital pathology algorithm, and 5) Human reader confirmation of CTCs & quantitation of biomarker expression. (Figure 1)

AR-V7 expression on Epic CTCs is 100% specific (100% PPV) to *de novo* PSA resistance, shorter time on drug (HR=4.61, p<0.0001), shorter rPFS (HR=2.92, p=0.0002), and shorter OS (HR=11.44 , p<0.0001) of patients receiving AR Therapy. Briefly, AR-V7 protein expression was found in CTCs from 15/66 patients resistant to AR Therapy. While CTCs were identified in 37/57 AR Therapy sensitive patients (range: is 0 to 345 CTCs/mL , median: 2 CTCs/mL), 0/57 harbored AR-V7+ CTCs. Of all patients in the AR Therapy cohort that lacked AR-V7+ cells, 53% were sensitive to AR Therapy. AR-V7 prevalence does not predict resistance to Taxane chemotherapy: AR-V7+ CTCs were found in 9/30 and 7/26 Taxane-sensitive and resistant patients, respectively. Figures 4 and 5.

AR-V7 expression is not associated with PSA resistance in taxane treated patients. Figure 4.

AR-V7 prevalence increases with increased exposure to systemic therapy AR-V7 (p <0.0001) and represents a minority population of total CTCs suggestive of disease heterogeneity. Figure 6.

This example demonstrates the association between the presence of AR-V7 positive CTCs and AR therapy resistance but not taxane resistance, confirming the utilization of the AR-V7 biomarker to inform treatment selection in mCRPC patients.

## Claims

1. A method of identifying a metastatic castration resistant prostate cancer (mCRPC) patient with an improved response to taxane therapy compared to androgen receptor signaling-directed (ARS-directed) therapy comprising:
(a) performing a direct analysis comprising immunofluorescent staining and morphological characterization of nucleated cells in a blood sample from the patient to generate circulating tumor cell (CTC) data,
wherein the direct analysis means the detection of the CTCs in the context of all surrounding nucleated cells present in the sample as opposed to after enrichment of the sample for CTCs prior to detection, and
wherein the direct analysis comprises detecting the presence of an Androgen Receptor Variant 7 (AR-V7) in said cells, and
(b) evaluating the CTC data to identify an mCRPC patient with an improved response to taxane therapy compared to ARS-directed therapy, wherein the presence of an Androgen Receptor Variant 7 in said CTCs identifies a mCRPC patient with an improved response to taxane therapy compared to ARS-directed therapy

2. The method of claim 1, wherein said mCRPC patient with an improved response to taxane therapy compared to ARS-directed therapy is identified based on nuclear localization of the AR-V7 in CTCs.

3. The method of claim 2, wherein the nuclear localization of the AR-V7 corresponds to resistance to ARS-directed therapy.

4. The method of claim 2, wherein the nuclear localization of the AR-V7 corresponds to an improved response to taxane therapy compared to ARS-directed therapy.

5. The method of claim 2, wherein said nuclear localization comprises a staining pattern with signal intensity ≥3-fold higher than background staining from neighboring white blood cells (WBCs).

6. The method of claim 1, further comprising an initial step of depositing the nucleated cells as a monolayer onto a slide.

7. The method of claim 1, wherein the direct analysis comprises fluorescent scanning microscopy.

8. The method of claim 7, wherein the microscopy provides a field of view comprising CTCs and at least 200 surrounding white blood cells (WBCs).

9. The method of claim 1, wherein CTCs comprise distinct morphological characteristics compared to surrounding nucleated cells.

10. The method of claim 9, wherein the morphological characteristics comprise one or more of the group consisting of nucleus size, nucleus shape, presence of holes in nucleus, cell size, cell shape and nuclear to cytoplasmic ratio, nuclear detail, nuclear contour, presence or absence of nucleoli, quality of cytoplasm and quantity of cytoplasm.

11. The method of claim 1, wherein the detection of CTCs further comprises comparing intensity of pan cytokeratin (CK) fluorescent staining to surrounding nucleated cells.

12. The method of claim 1, further comprising an initial step of obtaining a white blood cell (WBC) count for the blood sample.

13. The method of claim 1, further comprising an initial step of lysing erythrocytes in the blood sample.

14. The method of claim 1, wherein the immunofluorescent staining of nucleated cells to detect CTCs comprises pan cytokeratin (CK), cluster of differentiation (CD) 45, and diamidino-2-phenylindole (DAPI).

15. A taxane for use in a method of treating metastatic castration resistant prostate cancer (mCRPC), wherein the method comprises identifying a mCRPC patient with an improved response to taxane therapy compared to androgen receptor signaling-directed (ARS-directed) therapy as defined in any one of claims 1 to 14; and treating the patient with taxane therapy if the patient has been identified as having an improved response to taxane therapy compared to ARS-directed therapy.

## Patentansprüche

1. Verfahren zum Identifizieren eines Patienten mit metastasenbildendem, kastrationsresistentem Prostatakrebs (mCRPC) mit einem im Vergleich zu androgenrezeptorsignalisierungsgerichteter (ARS-gerichteter) Therapie besseren Ansprechen auf Taxantherapie, umfassend:
(a) Durchführen einer direkten Analyse, umfassend Immunofluoreszenzfärbung und morphologische Charakterisierung von kernhaltigen Zellen in einer Blutprobe des Patienten, um Daten zu zirkulierenden Tumorzellen (CTC) zu generieren,
wobei die direkte Analyse den Nachweis der CTCs im Kontext aller umgebenden kernhaltigen Zellen bedeutet, die in der Probe vorhanden sind, im Gegensatz zu nach Anreicherung der Probe mit CTCs vor dem Nachweis; und
wobei die direkte Analyse das Nachweisen des Vorhandenseins einer Androgenrezeptorvariante 7 (AR-V7) in den Zellen umfasst und
(b) Evaluieren der CTC-Daten, um einen mCRPC-Patienten mit einem im Vergleich zu ARS-gerichteter Therapie besseren Ansprechen auf Taxantherapie zu identifizieren, wobei das Vorhandensein einer Androgenrezeptorvariante 7 in den CTCs einen mCRPC-Patienten mit einem im Vergleich zu ARS-gerichteter Therapie besseren Ansprechen auf Taxantherapie identifiziert.

2. Verfahren nach Anspruch 1, wobei der mCRPC-Patient mit einem im Vergleich zu ARS-gerichteter Therapie besseren Ansprechen auf Taxantherapie auf Grundlage von Kernlokalisation der AR-V7 in CTCs identifiziert wird.

3. Verfahren nach Anspruch 2, wobei die Kernlokalisation der AR-V7 Resistenz gegenüber ARS-gerichteter Therapie entspricht.

4. Verfahren nach Anspruch 2, wobei die Kernlokalisation der AR-V7 einem im Vergleich zu ARS-gerichteter Therapie besseren Ansprechen auf Taxantherapie entspricht.

5. Verfahren nach Anspruch 2, wobei die Kernlokalisation ein Färbungsmuster mit ≥ 3-Mal höherer Signalintensität als Hintergrundfärbung von benachbarten weißen Blutzellen (WBCs) umfasst.

6. Verfahren nach Anspruch 1, weiter umfassend einen anfänglichen Schritt des Ablagerns der kernhaltigen Zellen als eine Monoschicht auf einen Objektträger.

7. Verfahren nach Anspruch 1, wobei die direkte Analyse Fluoreszenzabtastmikroskopie umfasst.

8. Verfahren nach Anspruch 7, wobei die Mikroskopie ein Sichtfeld bereitstellt, das CTCs und mindestens 200 umgebende weiße Blutzellen (WBCs) umfasst.

9. Verfahren nach Anspruch 1, wobei CTCs im Vergleich zu umgebenden kernhaltigen Zellen unterscheidbare morphologische Charakteristika umfassen.

10. Verfahren nach Anspruch 9, wobei die morphologischen Charakteristika eines oder mehrere umfassen von der Gruppe, bestehend aus Kerngröße, Kerngestalt, Vorhandensein von Löchern im Kern, Zellgröße, Zellgestalt und Kern-zu-Zytoplasma-Verhältnis, Kerndetail, Kernkontur, Vorhandensein oder Fehlen von Nukleoli, Qualität des Zytoplasmas und Quantität des Zytoplasmas.

11. Verfahren nach Anspruch 1, wobei der Nachweis von CTCs weiter das Vergleichen von Intensität von Pan-Zytokeratin (CK)-Fluoreszenzfärbung mit umgebenden kernhaltigen Zellen umfasst.

12. Verfahren nach Anspruch 1, weiter umfassend einen anfänglichen Schritt des Erhaltens einer Zählung weißer Blutzellen (WBCs) für die Blutprobe.

13. Verfahren nach Anspruch 1, weiter umfassend einen anfänglichen Schritt des Lysierens von Erythrozyten in der Blutprobe.

14. Verfahren nach Anspruch 1, wobei die Immunofluoreszenzfärbung von kernhaltigen Zellen, um CTCs nachzuweisen, Pan-Zytokeratin (CK),
Unterscheidungsgruppe (CD) 45 und Diamidin-2-phenylindol (DAPI) umfasst.

15. Taxan zur Verwendung bei einem Verfahren zum Behandeln von metastasenbildendem, kastrationsresistentem Prostatakrebs (mCRPC), wobei das Verfahren umfasst das Identifizieren eines mCRPC-Patienten mit einem im Vergleich zu androgenrezeptorsignalisierungsgerichteter (ARS-gerichteter) Therapie besseren Ansprechen auf Taxantherapie wie in einem der Ansprüche 1 bis 14 definiert; und Behandeln des Patienten mit Taxantherapie, wenn der Patient als ein im Vergleich zu ARS-gerichteter Therapie besseres Ansprechen auf Taxantherapie aufweisend identifiziert worden ist.

## Revendications

1. Procédé d'identification d'un patient atteint d'un cancer métastatique de la prostate résistant à la castration (CPRCm) ayant une meilleure réponse à un traitement par taxane qu'à un traitement ciblant la signalisation des récepteurs des androgènes (ciblant la SRA), comprenant :
(a) la réalisation d'une analyse directe comprenant une coloration par immunofluorescence et une caractérisation morphologique de cellules nucléées dans un échantillon de sang prélevé sur le patient pour générer des données sur les cellules tumorales en circulation (CTC),
dans lequel l'analyse directe signifie la détection des CTC dans le contexte de l'ensemble des cellules nucléées voisines présentes dans l'échantillon par opposition à après l'enrichissement de l'échantillon pour des CTC avant détection, et
dans lequel l'analyse directe comprend la détection de la présence d'une variante 7 du récepteur des androgènes (V7-RA) dans lesdites cellules, et
(b) l'évaluation des données sur les CTC pour identifier un patient atteint de CPRCm ayant une meilleure réponse à un traitement par taxane qu'à un traitement ciblant la SRA, dans lequel la présence d'une variante 7 du récepteur des androgènes dans lesdites CTC identifie un patient atteint de CPRCm ayant une meilleure réponse à un traitement par taxane qu'à un traitement ciblant la SRA

2. Procédé selon la revendication 1, dans lequel ledit patient atteint d'un CPRCm ayant une meilleure réponse à un traitement par taxane qu'à un traitement ciblant la SRA est identifié sur la base de la localisation nucléaire de la V7-RA dans les CTC.

3. Procédé selon la revendication 2, dans lequel la localisation nucléaire de la V7-RA correspond à la résistance à un traitement ciblant la SRA.

4. Procédé selon la revendication 2, dans lequel la localisation nucléaire de la V7-RA correspond à une meilleure réponse à un traitement par taxane qu'à un traitement ciblant la SRA

5. Procédé selon la revendication 2, dans lequel ladite localisation nucléaire comprend un motif de coloration avec une intensité de signal ≥ 3 fois supérieure à la coloration de fond provenant des leucocytes voisins.

6. Procédé selon la revendication 1, comprenant en outre une étape initiale de dépôt des cellules nucléées sous la forme d'une monocouche sur une lame.

7. Procédé selon la revendication 1, dans lequel l'analyse directe comprend une microscopie à balayage par fluorescence.

8. Procédé selon la revendication 7, dans lequel la microscopie fournit un champ de vue comprenant des CTC et au moins 200 leucocytes voisins.

9. Procédé selon la revendication 1, dans lequel des CTC comprennent des caractéristiques morphologiques distinctes par rapport à des cellules nucléées voisines.

10. Procédé selon la revendication 9, dans lequel les caractéristiques morphologiques comprennent une ou plusieurs du groupe constitué par la taille du noyau, la forme du noyau, la présence de trous dans le noyau, la taille de cellule, la forme de cellule et le rapport noyau/cytoplasme, le détail du noyau, le contour du noyau, la présence ou l'absence de nucléoles, la qualité du cytoplasme et la quantité de cytoplasme.

11. Procédé selon la revendication 1, dans lequel la détection de CTC comprend en outre la comparaison de l'intensité de la coloration par fluorescence de la pan cytokératine (CK) aux cellules nucléées voisines.

12. Procédé selon la revendication 1, comprenant en outre une étape initiale d'obtention d'une numération des leucocytes pour l'échantillon de sang.

13. Procédé selon la revendication 1, comprenant en outre une étape initiale de lyse d'érythrocytes dans l'échantillon de sang.

14. Procédé selon la revendication 1, dans lequel la coloration par immunofluorescence de cellules nucléées pour détecter des CTC comprend la pan cytokératine (CK), la classe de différenciation (CD) 45, et le diamidino-2-phénylindole (DAPI).

15. Taxane pour utilisation dans un procédé de traitement d'un cancer métastatique de la prostate résistant à la castration (CPRCm), dans lequel le procédé comprend l'identification d'un patient atteint d'un CPRCm ayant une meilleure réponse à un traitement par taxane qu'à un traitement ciblant la signalisation des récepteurs des androgènes (ciblant la SRA) selon l'une quelconque des revendications 1 à 14 ; et le traitement du patient par un traitement par taxane si le patient a été identifié comme ayant une meilleure réponse à un traitement par taxane qu'à un traitement ciblant la SRA
